# EUROPÄISCHE PATENTSCHRIFT

(11) **EP 1 304 131 B1**
(45) Veröffentlichungstag und Bekanntmachung des Hinweises auf die Patenterteilung: **29.06.2005**
(21) Anmeldenummer: 01124295.5
(22) Anmeldetag: 18.10.2001
(51) Int. Cl.: A61M 15/00, A61M 16/00

(54) **Inhalationstherapievorrichtung**
Inhalator
Appareil de ventilation

(43) Veröffentlichungstag der Anmeldung: 23.04.2003
(73) Patentinhaber: PARI GmbH Spezialisten für effektive Inhalation, 82319 Starnberg (DE)
(72) Erfinder: Feiner, Franz, 81735 München (DE); Borgschulte, Markus, 81453 München (DE)
(74) Vertreter: HOFFMANN - EITLE

## Beschreibung

Die Erfindung betrifft Inhalationstherapiegeräte mit einem Aerosolerzeuger, insbesondere mit einer schwingungsfähigen Membran zur Vernebelung einer Flüssigkeit oder eines Pulvers.

Die Steuerung von Inhalationstherapiegeräten erfolgt in vielen Fällen unter Berücksichtigung der Atmung des Patienten, um das Aerosol nur während der Einatmung zu erzeugen bzw. zur Einatmung bereit zu stellen. Auf diese Weise wird einerseits eine verbesserte Applikation des im Aerosol enthaltenen Medikaments und andererseits eine Verringerung der Medikamentenverluste ereicht. Für diese Form der Steuerung von Inhalationsgeräten ist es erforderlich, die Atmung des Patienten während der Inhalationstherapie zuverlässig zu erfassen. Dazu werden bei den bekannten Inhalationsverneblern Atemsensoren eingesetzt, die die durch die Atmung des Patienten hervorgerufenen Druckschwankungen erfassen und in ein Ausgangssignal umsetzten, das einer Steuereinrichtung des Inhalationsverneblers zugeführt, dort ausgewertet und in geeignete Steuervorgänge umgesetzt wird.

Durch die Erfindung wird ein Weg aufgezeigt, wie die Steuerung einer Inhalationstherapievorrichtung in Abhängigkeit von der Atmung des Patienten erfolgen kann, ohne dass ein separater Atemsensor verwendet wird. Dadurch wird der Aufwand bei der Herstellung des Inhalationsverneblers verringert und dessen Störanfälligkeit herabgesetzt.

Erfindungsgemäß wird dies erreicht durch eine Inhalationstherapievorrichtung mit einer schwingungsfähigen Membran für die Vernebelung einer Flüssigkeit, mit einer Schwingungserzeugungseinrichtung, die zumindest eine Anschlusseinrichtung für die Zuführung eines Ansteuerungssignals aufweist und durch die die Membran in Schwingungen versetzt wird, wenn das Ansteuerungssignal zugeführt wird, so dass eine auf einer Seite der Membran anstehende Flüssigkeit durch die Membran hindurch vernebelt wird und auf der anderen Seite der Membran als Aerosol vorliegt, und mit einer Steuereinrichtung, von der ein Ansteuerungssignal der zumindest einen Anschlusseinrichtung der Schwingungserzeugungseinrichtung zuführbar ist, so dass die Schwingungserzeugungseinrichtung die Membran in Schwingungen versetzt, wobei erfindungsgemäß ein durch die Atmung des Patienten an der zumindest einen Anschlusseinrichtung der Schwingungserzeugungseinrichtung hervorgerufenes Ausgangssignal der Steuereinrichtung zur Steuerung der Inhalationstherapievorrichtung zugeführt wird.

Die Erfindung beruht auf der durchaus überraschenden Erkenntnis, dass bei Membranverneblern die für die Schwingungserzeugung eingesetzte elektro-mechanische Wandlereinheit, in der Regel ein piezoelektrisches Bauteil, ein atmungsabhängiges Ausgangssignal bereitstellt, das für die Steuerung des Inhalationstherapiegeräts herangezogenen werden kann, und zwar unabhängig davon, ob der Membranvernebler angesteuert wird oder nicht.

Somit wird durch die erfindungsgemäße Gestaltung der Vorteil erreicht, dass ein Atemsensor der oben erwähnten Art nicht mehr erforderlich ist. Denn bei einem erfindungsgemäßen Inhalationstherapiegerät kann auf einen separaten Atemsensor verzichtet werden, da ein auf die Patientenatmung zurückgehendes Ausgangssignal des Aerosolerzeugers verwendet wird, um die Ansteuerung des Aerosolerzeugers zu steuern.

Grundsätzlich eignet sich die erfindungsgemäße Lösung für alle Inhalationstherapiegeräte, bei denen ein Aerosolerzeuger zum Einsatz kommt, der ein atmungsabhängiges Ausgangssignal erzeugt, das abgegriffen, verarbeitet, insbesondere von dem Ansteuerungssignal getrennt und für die Steuerung ausgewertet werden kann. Damit ist die Anwendbarkeit der Erfindung nicht auf Membranvernebler beschränkt, obwohl Vernebler dieser Art sich besonders vorteilhaft eignen.

Damit kann die Erfindung auch zusammengefasst charakterisiert werden als Inhalationstherapievorrichtung mit einer Aerosolerzeugungseinrichtung für die Vernebelung einer Flüssigkeit oder eines Pulvers, die eine Anschlusseinrichtung für die Zuführung eines Ansteuerungssignals aufweist, und mit einer Steuereinrichtung, von der ein Ansteuerungssignal der Anschlusseinrichtung der Aerosolerzeugungseinrichtung zuführbar ist, so dass die Aerosolerzeugungseinrichtung die Flüssigkeit vernebelt, bei der ein Ausgangssignal, das durch die Atmung des Patienten an der Anschlusseinrichtung
der Aerosolerzeugungseinrichtung hervorgerufen wird, der Steuereinrichtung zur Steuerung der Inhalationstherapievorrichtung zugeführt wird.

Von Vorteil ist bei der erfindungsgemäßen Lösung auch, dass zusätzliche Elektroden oder andere zusätzliche Signalabgriffe oder -ableitungen nicht erforderlich sind. Denn die Ableitung des Atemsignals erfolgt vorzugsweise über die gegebenen Elektroden bzw. Verbindungsleitungen; ein Eingriff in die Gestaltung der elektro-mechanischen Wandlereinheit oder die Gestaltung des Inhalationsverneblers ist nicht nötig.

Der Umstand, dass in den Inhalationsvernebler und in dessen Aerosolerzeuger nicht konstruktiv eingegriffen werden muss, führt auch dazu, dass vorhandene Inhalationstherapiegeräte nachgerüstet werden können. Denn um ein vorhandenes Inhalationstherapiegerät mit den erfindungsgemäß möglichen Steuerungsfunktionalitäten auszustatten, reicht es aus, dessen Steuerungseinheit, die in der Regel als separates Gerät realisiert ist, gegen eine Steuerungseinheit auszutauschen, bei der die Auswertung des erfindungsgemäßen Atmungssignals erfolgt.

Im Hinblick auf die zuvor beschriebene Nachrüstung vorhandener Geräte aber auch grundsätzlich ist es vorteilhaft, wenn die Verarbeitung des Ausgangssignals in derselben baulichen Einheit oder sogar innerhalb derselben elektrischen Einheit erfolgt, in der auch die Steuereinrichtung realisiert ist, da so zusätzliche Verbindungsleitungen zur Ableitung des Atmungssignals vermieden werden. Dies ist insbesondere bei Inhalationstherapiegeräten von Bedeutung, bei denen die Steuerung und andere Komponenten, beispielsweise die Stromversorgung, in einem vom Vernebler getrennten Gehäuse untergebracht sind. Bei Anwendung der Erfindung ist es für die Verbindung zwischen Vernebler und Steuereinheit ausreichend, die für die Übertragung des Ansteuerungssignals zum Aerosolerzeuger erforderlichen Leitungsverbindungen vorzusehen. Das Abgreifen des durch die Patientenatmung hervorgerufenen Ausgangssignals kann ebenfalls über diese Verbindungsleitungen erfolgen.

Im folgenden wird die Erfindung anhand eines Ausführungsbeispiels unter Bezugnahme auf die Figuren genauer erläutert, in denen zeigt:
- Fig. 1: eine schematische Darstellung einer erfindungsgemäßen Inhalationstherapievorrichtung; und
- Fig. 2: ein Blockschaltbild der Verarbeitungseinheit der erfindungsgemäßen Inhalationstherapievorrichtung aus Fig. 1.

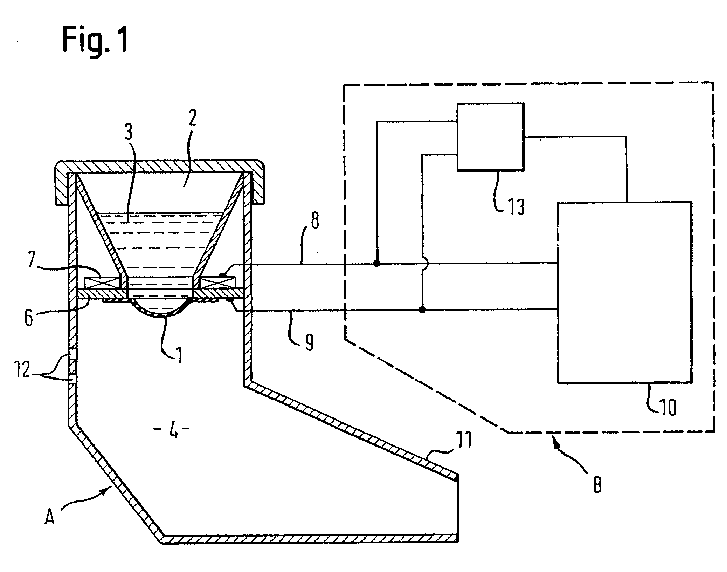

Fig. 1 zeigt ein Ausführungsbeispiel einer erfindungsgemäßen Inhalationstherapievorrichtung, bei der in einer Verneblereinheit A mit Hilfe einer Membran 1 eine in einem Flüssigkeitsreservoir 2 bevorratete Flüssigkeit 3 in einen Vernebelungshohlraum 4 hinein vernebelt wird. Die Vernebelung findet dann statt, wenn die Membran 1 in Schwingungen versetzt wird. Dazu ist die Membran 1 an einer Supporteinheit 6 befestigt, die die Membran 1 trägt und an der auch eine elektro-mechanische Wandlereineinheit 7, beispielsweise ein Piezoelement, befestigt ist. Die Membran 1, die Supporteinheit 6 und die elektro-mechanische Wandlereinheit 7 sind bei dem hier beschriebenen Ausführungsbeispiel rotationssymmetrisch ausgebildet und bilden zusammen ein schwingungsfähiges Gebilde. Über Anschlussleitungen 8 und 9 kann an die elektro-mechanische Wandlereinheit 7 ein Ansteuerungssignal von einer Steuereinrichtung 10 zugeführt werden, die bei dem hier beschriebenen Ausführungsbeispiel in einer separaten Steuerungseinheit B untergebracht ist. Wenn das Ansteuerungssignal zugeführt wird, wird das schwingfähige Gebilde 1, 6, 7 in Schwingungen versetzt und die Flüssigkeit 3 durch die Membran 1 hindurch vernebelt.

Ein Patient kann das im Vernebelungshohlraum 4 bereitgestellte Aerosol am Mundstück 11 des Verneblers einatmen. Für die Zuführung einer ausreichenden Atemluftmenge sind im Gehäuse des Verneblers ein oder mehrere Atemöffnungen 12 vorgesehen, durch die Umgebungsluft in den Hohlraum 4 während des Einatmens eintreten und aus denen die Atemluft des Patienten aus dem Hohlraum 4 während des Ausatmens austreten kann. Im Vergleich mit der Umgebung außerhalb des Hohlraums 4 treten im Hohlraum 4 beim Einatmen und beim Ausatmen Druckschwankungen auf. Beim Einatmen sinkt der Druck in der Verneblerkammer 4 unter den Umgebungsdruck, beim Ausatmen steigt er über den Umgebungsdruck an. Zwar findet durch die Atemöffnungen 12 ein Druckausgleich statt, jedoch wurde durch Untersuchungen, die zu der hier beschriebenen Erfindung geführt haben, festgestellt, dass die Druckschwankungen in der Regel ausreichend sind um auf die Membran 1 derart einzuwirken, dass ein verwertbares atmungsabhängiges Ausgangssignal von der elektro-mechanischen Wandlereinheit 7 abgegeben wird. Durch die Gestaltung der Atemöffnungen 12 und vorzugsweise zusätzlich durch entsprechend gestaltete Ventilelemente an den Atemöffnungen 12 kann die Höhe der Druckschwankungen und damit die Stärke der Einwirkung der Patientenatmung auf die Membran 1 beeinflusst werden.

Bei dem gezeigten Ausführungsbeispiel steht ein von der Patientenatmung hervorgerufenes Ausgangssignal an den beiden Anschlussleitungen 8 und 9 zur Verfügung, die auch zur Zuführung des Ansteuerungssignals zu dem schwingungsfähigen Gebilde 1, 6, 7 verwendet werden. Da die Anregung der Membran 1 mit einem Signal erfolgt, dessen Frequenz sehr viel größer ist als die Atemfrequenz eines Menschen, kann an dem Ansteueranschluss 8, 9 des schwingungsfähigen Gebildes 1, 6, 7 das Ausgangssignal abgegriffen werden, das auf die durch die Atmung des Patienten hervorgerufenen Druckschwankungen im Vernebelungsraum 4 zurückgeht. Erfindungsgemäß wird dieses Atmungssignal der Steuereinheit 10 zugeführt und dort bei der Ansteuerung der Inhalationstherapievorrichtung berücksichtigt.

Wie bei dem hier beschriebenen Ausführungsbeispiel gezeigt, ist vorzugsweise eine geeignete Verarbeitungseinheit 13 vorgesehen, die als getrennte Einheit, beispielsweise eine Filter-/VerstärkerSchaltung ausgelegt ist, und die das von der Patientenatmung beeinflusste Ausgangssignal an den Anschlüssen des schwingungsfähigen Gebildes 1, 6, 7, also des Aerosolerzeugers, zu einem Steuerungssignal verarbeitet, das dem Atemzyklus des Patienten folgt. Dieses Signal wird von der Steuereinrichtung 10 verwertet, indem die Ansteuerung des schwingungsfähigen Gebildes 1, 6, 7 und damit die Vernebelung der Flüssigkeit 3 durch die Membran 1 nur in den gewünschten Abschnitten des Atemzyklus, beispielsweise nur während des Einatmens erfolgt.

Auf diese Weise lässt sich erfindungsgemäß zunächst ein einfacher Ein/Aus-Schaltvorgang realisieren, d.h die Vernebelung beginnt mit dem Einsetzen der Einatmung und endet am Ende der Einatmung. Jedoch wurde bei den auf der Erfindung beruhenden Untersuchungen festgestellt, dass das erfindungsgemäß verwertbare Atmungssignal des schwingungsfähigen Gebildes 1, 6, 7 wesentlich genauere Aussagen über den Atemzyklus des Patienten erlaubt, und zwar auch wenn über die Anschlussleitungen 8 und 9 das Ansteuerungssignal dem schwingungsfähige Gebilde 1, 6, 7 zugeführt wird. Mit anderen Worten, die beiden Signale, nämlich das Ansteuerungssignal der Steuereinrichtung 10, durch das das schwingungsfähige Gebilde 1, 6, 7 in Schwingungen versetzt wird, und das Ausgangssignal des schwingungsfähigen Gebildes 1, 6, 7, das durch die Patientenatmung hervorgerufen wird, überlagern einander quasi störungsfrei. Dies ist durchaus überraschend, da das durch die Druckschwankungen hervorgerufene Ausgangssignal an der Anschlusseinrichtung 8, 9 des schwingungsfähigen Gebildes 1, 6, 7 sehr viel kleiner ist als das Ansteuerungssignal, das von der Steuervorrichtung 10 über die Anschlussleitungen 8 und 9 an das schwingungsfähige Gebilde 1, 6, 7 herangeführt wird. Damit können aber bei Anwendung der Erfindung dem Patienten optische oder akustische Hinweise zur Optimierung des Atemflusses gegeben werden oder es kann eine automatische Abschaltung der Aerosolerzeugung erfolgen, wenn zu hohe Atemflüsse festgestellt werden, die für eine sichere intrapulmonale Aerosoldeposition ungünstig sind.

Um eine zuverlässige Trennung von Atmungssignal und Ansteuerungssignal zu gewährleisten, ist vorzugsweise die Steuereinrichtung 10 derart ausgelegt, dass zur Ansteuerung des schwingungsfähigen Gebildes 1, 6, 7 für die Vernebelung der Flüssigkeit 3 keine niederfrequenten Signalkomponenten, insbesondere keine Gleichspannungskomponenten verwendet werden. Durch diese Gestaltung wird auch erreicht, dass das durch die Patientenatmung hervorgerufene Ausgangsignal von dem Ansteuerungssignal mit vergleichsweise geringem Aufwand und damit kostengünstig getrennt werden kann.

Das oben genauer beschriebene Ausführungsbeispiel zeigt, wie die erfindungsgemäße Nutzung des Atmungssignal bei einem Inhalationstherapiegerät mit Membranvernebler erfolgt. Die Beschreibung des Ausführungsbeispiels macht aber auch deutlich, dass die Erfindung bei allen Inhalationstherapiegeräten angewendet werden kann, bei denen eine Aerosolerzeugungseinrichtung mit einem Ansteuerungssignal beaufschlagt wird, um ein Aerosol zu erzeugen, und bei denen die Atmung des Patienten auf die Aerosolerzeugungseinrichtung derart einwirkt, dass ein Ausgangssignal zur Verfügung steht, das der Steuereinrichtung zur Ansteuerung der Aerosolerzeugungseinrichtung zugeführt werden kann.

Vorrangig einsetzbar ist die Erfindung bei solchen Inhalationstherapiegeräten, bei denen eine elektro-mechanische Wandlereinheit vorgesehen ist, die ein die Erzeugung des Aerosols bewirkendes schwingfähiges Gebilde in Schwingungen versetzt. Auf das schwingfähige Gebilde wirken regelmäßig die durch die Patientenatmung hervorgerufenen Druckschwankungen und führen zu den Druckschwankungen entsprechende Bewegungen des schwingungsfähigen Gebildes. Diese auf die Druckschwankungen zurückgehenden Bewegungen rufen ein Ausgangssignal hervor, das aufgrund der Bewegung von der elektro-mechanischen Wandlereinheit erzeugt wird.

In besonderem Maße eignen sich zur Anwendung der Erfindung Inhalationstherapiegeräte, bei denen die elektro-mechanische Wandlereinheit in Form eines piezoelektrischen Elements ausgebildet ist.

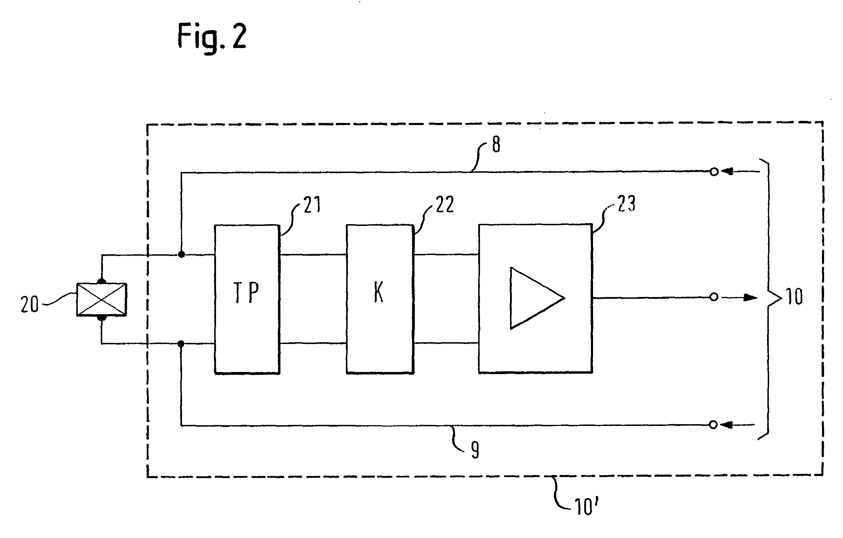

Wie in Fig. 2 dargestellt, umfasst die Verarbeitungseinheit 13 des hier beschriebenen Ausführungsbeispiels die im folgenden beschriebenen Untereinheiten. Zur Vereinfachung der Darstellung in Fig. 2 ist das schwingungsfähige Gebilde, bestehend aus Membran 1, Supportelement 6 und Piezoelement 7, in Fig. 2 ersetzt worden durch eine schematische Darstellung eines elektro-mechanischen Piezo-Wandlers 20. Das an der Anschlusseinrichtung abgegriffene atmungsbeeinflusste Ausgangssignal des Piezo-Wandlers 20 wird in der Verarbeitungseinheit 13 einer Tiefpasseinheit 21 zugeführt, die zunächst die hohen Frequenzkomponenten des abgegriffenen Signals und damit insbesondere die durch das Ansteuerungssignal verursachten Komponenten des abgegriffenen Signals ausfiltert. Das Ausgangssignal des Tiefpasses 21 wird einer Kopplungseinheit 22, beispielsweise gebildet durch Kondensatoren geeigneter Kapazität zugeführt. Das Ausgangssignal der Kopplungseinheit 22 wird einer Verstärkereinheit 23 zugeführt, die das zugeführte Signal in ein für die Steuereinheit 10 geeignetes Ausgangssignal umwandelt.

Wie in Fig. 2 durch Pfeile angedeutet, wird das durch die Verarbeitungseinheit 13 verarbeitete Atmungssignal der Steuereinrichtung 10 zugeführt, von der das Ansteuerungssignal für den Piezo-Wandler 20 über Anschlussleitungen8 und 9 zugeführt wird. Durch die gestrichelte Linie in Fig. 2 ist angedeutet, dass die Steuereinrichtung 10 und die Verarbeitungseinrichtung 13 gemeinsam in einem Gehäuse oder sogar in einer Schaltung / einem Schaltkreis angeordnet werden können.

## Patentansprüche

1. Inhalationstherapievorrichtung
a. mit einer schwingungsfähigen Membran (1) für die Vernebelung einer Flüssigkeit (3),
b. mit einer Schwingungserzeugungseinrichtung (6,7), die zumindest eine Anschlusseinrichtung (8,9) für die Zuführung eines Ansteuerungssignals aufweist und durch die die Membran (1) in Schwingungen versetzt wird, wenn das Ansteuerungssignal zugeführt wird, so dass eine auf einer Seite der Membran anstehende Flüssigkeit durch die Membran hindurch vernebelt wird und auf der anderen Seite der Membran als Aerosol vorliegt, und
c. mit einer Steuereinrichtung (10), von der ein Ansteuerungssignal der zumindest einen Anschlusseinrichtung (8,9) der Schwingungserzeugungseinrichtung (6,7) zuführbar ist, so dass die Schwingungserzeugungseinrichtung (6,7) die Membran (1) in Schwingungen versetzt,
**dadurch gekennzeichnet, dass**
ein durch die Atmung eines Patienten an der zumindest einen Anschlusseinrichtung (8,9) der Schwingungserzeugungseinrichtung (6,7) hervorgerufenes Ausgangssignal der Steuereinrichtung (10) zur Steuerung der Inhalationstherapievorrichtung zugeführt wird.

2. Inhalationstherapievorrichtung nach Anspruch 1, **dadurch gekennzeichnet, dass** die Schwingungserzeugungseinrichtung (6, 7) eine elektro-mechanische Wandlereinheit (7), insbesondere ein piezo-elektrisches Element umfasst.

3. Inhalationstherapievorrichtung nach Anspruch 2, **dadurch gekennzeichnet, dass** die Schwingungserzeugungseinrichtung (6, 7) eine Supporteinheit (6) umfasst, an der die elektro-mechanische Wandlereinheit (6) und die Membran (1) befestigt sind.

4. Inhalationstherapievorrichtung nach einem der vorangegangenen Ansprüche, **dadurch gekennzeichnet, dass** eine Verarbeitungseinheit (13) vorgesehen ist, über die das Ausgangssignal an der zumindest einen Anschlusseinrichtung (8,9) der Schwingungserzeugungseinrichtung (6,7) der Steuereinrichtung (10) zugeführt wird und die ein Atmungssignal bereitstellt, das dem Verlauf der Patientenatmung folgt.

5. Inhalationstherapievorrichtung nach Anspruch 4, **dadurch gekennzeichnet, dass** die Verarbeitungseinheit (13) eine Tiefpasseinheit (21), eine Kopplungseinheit (22) und eine Verstärkereinheit (23) umfasst.

6. Inhalationstherapievorrichtung nach Anspruch 4 oder 5, **dadurch gekennzeichnet, dass** die Verarbeitungseinheit (13) in die Steuereinrichtung (10) integriert ist.

7. Inhalationstherapievorrichtung nach einem der vorangegangenen Ansprüche, **dadurch gekennzeichnet, dass** in der Steuereinrichtung (10) eine Energieversorgungseinrichtung für die Inhalationsvorrichtung integriert ist.

8. Inhalationstherapievorrichtung
a. mit einer Aerosolerzeugungseinrichtung (1,6,7) für die Vernebelung einer Flüssigkeit (3) oder eines Pulvers, die eine Anschlusseinrichtung (8,9) für die Zuführung eines Ansteuerungssignals aufweist, und
b. mit einer Steuereinrichtung (10), von der ein Ansteuerungssignal der Anschlusseinrichtung (8,9) der Aerosolerzeugungseinrichtung (1, 6, 7) zuführbar ist, so dass die Aerosolerzeugungseinrichtung die Flüssigkeit bzw. das Pulver vernebelt,
**dadurch gekennzeichnet, dass**
ein durch die Atmung eines Patienten an der Anschlusseinrichtung (8,9) der Aerosolerzeugungseinrichtung (1,6,7) hervorgerufenes Ausgangssignal der Steuereinrichtung (10) zur Steuerung der Inhalationstherapievorrichtung zugeführt wird.

9. Inhalationstherapievorrichtung nach Anspruch 8, **dadurch gekennzeichnet, dass** die Aerosolerzeugungseinrichtung ein schwingfähiges Gebilde (1,6,7) umfasst, auf das die Atmung des Patienten einwirkt, so dass ein atmungsabhängiges Ausgangssignal hervorgerufen wird.

10. Inhalationstherapievorrichtung nach Anspruch 8 oder 9, **dadurch gekennzeichnet, dass** die Aerosolerzeugungseinrichtung eine elektro-mechanische Wandlereinheit (7), vorzugsweise ein piezo-elektrisches Element umfasst.

11. Inhalationstherapievorrichtung nach Anspruch 8, 9 oder 10, **dadurch gekennzeichnet, dass** eine Verarbeitungseinheit (13) vorgesehen ist, über die das Ausgangssignal der Aerosolerzeugungseinrichtung (1,6,7) der Steuereinrichtung (10) zugeführt wird und die ein Atmungssignal bereitstellt, das dem Verlauf der Patientenatmung folgt.

12. Inhalationstherapievorrichtung nach Anspruch 11, **dadurch gekennzeichnet, dass** die Verarbeitungseinheit (13) eine Tiefpasseinheit (21), eine Kopplungseinheit (22) und eine Verstärkereinheit (23) umfasst.

13. Inhalationstherapievorrichtung nach Anspruch 11 oder 12, **dadurch gekennzeichnet, dass** die Verarbeitungseinheit (13) in die Steuereinrichtung (10) integriert ist.

14. Inhalationstherapievorrichtung nach einem der vorangegangenen Ansprüche, **dadurch gekennzeichnet, dass** die Steuereinrichtung (10) in eine Energieversorgungseinrichtung für die Inhalationstherapievorrichtung integriert ist.

## Claims

1. Inhalation treatment apparatus
a) with a vibratable membrane (1) for the atomisation of a liquid (3),
b) with a vibration-generating device (6, 7) which has at least one connection device (8, 9) for the delivery of a trigger signal and by which the membrane (1) is set in vibration when the trigger signal is delivered, so that a liquid which is on one side of the membrane is atomised through the membrane and is present as an aerosol on the other side of the membrane, and
c) with a control device (10) from which a trigger signal can be delivered to the at least one connection device (8, 9) of the vibration-generating device (6, 7), so that the vibration-generating device (6, 7) sets the membrane (1) in vibration,
**characterised in that** an output signal caused by the breathing of a patient at the at least one connection device (8, 9) of the vibration-generating device (6, 7) is delivered to the control device (10) for control of the inhalation treatment apparatus.

2. Inhalation treatment apparatus according to claim 1, **characterised in that** the vibration-generating device (6, 7) includes an electromechanical transducer unit (7), in particular a piezoelectric element.

3. Inhalation treatment apparatus according to claim 2, **characterised in that** the vibration-generating device (6, 7) includes a support unit (6) to which the electromechanical transducer unit (6) and the membrane (1) are attached.

4. Inhalation treatment apparatus according to any of the preceding claims, **characterised in that** there is provided a processing unit (13) via which the output signal at the at least one connection device (8, 9) of the vibration-generating device (6, 7) is delivered to the control device (10) and which provides a breathing signal which follows the curve of patient breathing.

5. Inhalation treatment apparatus according to claim 4, **characterised in that** the processing unit (13) includes a low-pass filter unit (21), a coupling unit (22) and an amplifier unit (23).

6. Inhalation treatment apparatus according to claim 4 or 5, **characterised in that** the processing unit (13) is integrated in the control device (10).

7. Inhalation treatment apparatus according to any of the preceding claims, **characterised in that** in the control device (10) is integrated a power supply device for the inhalation apparatus.

8. Inhalation treatment apparatus
a) with an aerosol-generating device (1, 6, 7) for the atomisation of a liquid (3) or powder, which has a connection device (8, 9) for the delivery of a trigger signal, and
b) with a control device (10) from which a trigger signal can be delivered to the connection device (8, 9) of the aerosol-generating device (1, 6, 7), so that the aerosol-generating device atomises the liquid or powder,
**characterised in that**
an output signal caused by the breathing of a patient at the connection device (8, 9) of the aerosol-generating device (1, 6, 7) is delivered to the control device (10) for control of the inhalation treatment apparatus.

9. Inhalation treatment apparatus according to claim 8, **characterised in that** the aerosol-generating device includes a vibratable structure (1, 6, 7) upon which the breathing of a patient acts so that a breathing-related output signal is caused.

10. Inhalation treatment apparatus according to claim 8 or 9, **characterised in that** the aerosol-generating device includes an electromechanical transducer unit (7), preferably a piezoelectric element.

11. Inhalation treatment apparatus according to claim 8, 9 or 10, **characterised in that** there is provided a processing unit (13) via which the output signal of the aerosol-generating device (1, 6, 7) is delivered to the control device (10) and which provides a breathing signal which follows the curve of patient breathing.

12. Inhalation treatment apparatus according to claim 11, **characterised in that** the processing unit (13) includes a low-pass filter unit (21), a coupling unit (22) and an amplifier unit (23).

13. Inhalation treatment apparatus according to claim 11 or 12, **characterised in that** the processing unit (13) is integrated in the control device (10).

14. Inhalation treatment apparatus according to any of the preceding claims, **characterised in that** the control device (10) is integrated in a power supply device for the inhalation treatment apparatus.

## Revendications

1. Dispositif thérapeutique d'inhalation
a) avec une membrane pouvant être mise en vibration (1) pour la nébulisation d'un liquide (3),
b) avec un dispositif de génération de vibrations (6, 7) qui présente au moins un dispositif de raccordement (8, 9) pour l'amenée d'un signal de commande, et par laquelle la membrane (1) est mise en vibration quand le signal de commande est amené, de manière à ce qu'un liquide, présent d'un côté de la membrane, soit nébulisé à travers ladite membrane et se présente sous la forme d'un aérosol, de l'autre côté de la membrane, et
c) avec un dispositif de commande (10), à partir de duquel un signal de commande peut être lancé à un dispositif de raccordement (8, 9) au moins prévu du dispositif de génération de vibrations (6, 7), de manière à ce que ledit dispositif de génération de vibrations (6, 7) mette la membrane (1) en vibration,
**caractérisé en ce que**
un signal de sortie, provoqué par la respiration d'un patient dans le dispositif de raccordement (8, 9) au moins prévu du dispositif de génération de vibrations, est conduit au dispositif de commande (10) pour la commande du dispositif thérapeutique d'inhalation.

2. Dispositif thérapeutique d'inhalation selon la revendication 1, **caractérisé en que** le dispositif de génération de vibrations (6, 7) comprend une unité de transduction électromécanique (7), notamment un élément piézoélectrique.

3. Dispositif thérapeutique d'inhalation selon la revendication 2, **caractérisé en que**
le dispositif de génération de vibrations (6, 7) comprend une unité de support (8) à laquelle l'unité de transduction électromécanique (7) et la membrane (1) peuvent être fixées.

4. Dispositif thérapeutique d'inhalation selon l'une des revendications précédentes, **caractérisé en que**
une unité de traitement (13) est prévue, par l'intermédiaire de laquelle le signal de sortie, lancé à un dispositif de raccordement (8, 9) au moins prévu du dispositif de génération de vibrations (6, 7) est conduit au dispositif de commande (10), et qui génère un signal de respiration qui suit l'évolution de la respiration du patient.

5. Dispositif thérapeutique d'inhalation selon la revendication 4, **caractérisé en que**
l'unité de traitement (13) comprend une unité passe-bas (21), une unité de couplage (22) et une unité d'amplification (23).

6. Dispositif thérapeutique d'inhalation selon la revendication 4 ou 5, **caractérisé en que**
l'unité de traitement (13) est intégrée dans le dispositif de commande (10).

7. Dispositif thérapeutique d'inhalation selon l'une des revendications précédentes, **caractérisé en que**, dans le dispositif de commande (10), est intégré un système d'alimentation en énergie pour le dispositif d'inhalation.

8. Dispositif thérapeutique d'inhalation
a) avec un système de génération d'aérosol (1, 6, 7) pour la nébulisation d'un liquide (3) ou d'une poudre, lequel présente un dispositif de raccordement (8, 9) pour l'arrivée d'un signal de commande, et
b) avec un dispositif de commande (10), à partir duquel un signal de commande peut être conduit au dispositif de raccordement (8, 9) du système de génération d'aérosol (1, 6, 7), de manière à ce que ledit système de génération d'aérosol nébulise le liquide resp. la poudre.
**caractérisé en ce que**
un signal de sortie, provoqué par la respiration d'un patient dans le dispositif de raccordement (8, 9) du système de génération d'aérosol (1, 6, 7), est conduit au dispositif de commande (10) pour la commande du dispositif thérapeutique d'inhalation.

9. Dispositif thérapeutique d'inhalation selon la revendication 8, **caractérisé en que**
le système de génération d'aérosol comprend un arrangement pouvant être mis en vibration (1, 6, 7), sur lequel agit la respiration du patient de telle manière qu'un signal de sortie est généré en dépendance de la respiration.

10. Dispositif thérapeutique d'inhalation selon la revendication 8 ou 9, **caractérisé en que**
le système de génération d'aérosol comprend une unité de transduction électromécanique (7), qui est préférentiellement un élément piézoélectrique.

11. Dispositif thérapeutique d'inhalation selon la revendication 8, 9 ou 10, **caractérisé en que**
une unité de traitement (13) est prévue, par l'intermédiaire de laquelle le signal de sortie du système de génération d'aérosol (1, 6, 7) est conduit au dispositif de commande (10), et qui génère un signal de respiration qui suit l'évolution de la respiration du patient.

12. Dispositif thérapeutique d'inhalation selon la revendication 11, **caractérisé en que**
l'unité de traitement (13) comprend une unité de passe-bas (21), une unité de couplage (22) et une unité d'amplification (23).

13. Dispositif thérapeutique d'inhalation selon la revendication 11 ou 12, **caractérisé en que**
l'unité de traitement (13) est intégrée dans le dispositif de commande (10).

14. Dispositif thérapeutique d'inhalation selon l'une des revendications précédentes, **caractérisé en que**
le dispositif de commande (10) est intégré dans une installation d'alimentation en énergie pour le dispositif thérapeutique d'inhalation.
